# EUROPÄISCHE PATENTSCHRIFT

(11) **EP 1 392 650 B1**
(45) Veröffentlichungstag und Bekanntmachung des Hinweises auf die Patenterteilung: **02.03.2005**
(21) Anmeldenummer: 02740662.8
(22) Anmeldetag: 29.05.2002
(51) Int. Cl.: C07C 309/30, C07C 227/18, C07B 59/00, C07M 5/00

(54) **GESCHÜTZTE TYROSINDERIVATE, VERFAHREN ZU DEREN HERSTELLUNG UND DEREN VERWENDUNG ZUR HERSTELLUNG VON O-(2- ?18 F]-FLUORETHYL)-L-TYROSIN**
PROTECTED TYROSINE DERIVATIVES, METHOD FOR THE PRODUCTION THEREOF AND USE OF THE SAME FOR PRODUCING O-(2- ?18 F]-FLUOROETHYL)-L-TYROSINE
DERIVES DE TYROSINE PROTEGES, LEUR PROCEDE DE PRODUCTION ET LEUR UTILISATION POUR PRODUIRE DE L'O-(2- ?18 F]-FLUORETHYL)-L-TYROSINE

(30) Priorität: 05.06.2001 DE 10127126
(43) Veröffentlichungstag der Anmeldung: 03.03.2004
(73) Patentinhaber: FORSCHUNGSZENTRUM JÜLICH GMBH, 52428 Jülich (DE)
(72) Erfinder: HAMACHER, Kurt, 52076 Aachen (DE)
(74) Vertreter: Gille Hrabal Struck Neidlein Prop Roos
(86) Internationale Anmeldenummer: PCT/EP2002/005887
(87) Internationale Veröffentlichungsnummer: WO 2002/102765

(56) Entgegenhaltungen:
- WO-A-97/31907
- WO-A-99/06431
- LI, LINTONG ET AL: "The tethered agonist approach to mapping ion channel proteins - toward a structural model for the agonist binding site of the nicotinic acetylcholine receptor" CHEMISTRY & BIOLOGY (2001), 8(1), 47-58 , XP002231648
- WESTER, HANS J. ET AL: "Synthesis and radiopharmacology of O-(2-[18F]fluoroethyl)-L-tyrosine for tumor imaging" JOURNAL OF NUCLEAR MEDICINE (1999), 40(1), 205-212 , XP001109689 in der Anmeldung erwähnt
- SINDONA G ET AL: "Simple Total Syntheses of N-Substituted Polyamine Derivatives Using N-Tritylamino Acids" TETRAHEDRON LETTERS, ELSEVIER SCIENCE PUBLISHERS, AMSTERDAM, NL, Bd. 36, Nr. 29, 17. Juli 1995 (1995-07-17), Seiten 5187-5190, XP004027637 ISSN: 0040-4039
- FURUMOTO, SHOZO ET AL: "Synthesis of 1-O-(8-[18F]fluorooctanoyl)-2-O-palmitoyl- rac-glycerol for imaging intracellular signal transduction" JOURNAL OF LABELLED COMPOUNDS & RADIOPHARMACEUTICALS ( 2000 ), 43(12), 1159-1172 , XP008013950

## Beschreibung

Die Erfindung betrifft ein Verfahren zur Herstellung von O-(2-[¹⁸F]-Fluorethyl)-L-tyrosin und neue Ausgangsverbindungen zur Herstellung von O-(2-[¹⁸F]-Fluorethyl)-L-tyrosin

Die Diagnose von Hirntumoren wird in der letzten Zeit zunehmend mittels des Positronen-Emissions-Tomographie (PET)-Verfahrens durchgeführt.

Anfangs haben die Untersuchungen mit dem PET-Verfahren sich auf die Untersuchung des Glukosestoffwechsels konzentriert, wobei als Diagnostikum die Verbindung ¹⁸F-fluor-Deoxyglukose (FDG) verwendet wurde. Mit dieser markierten Verbindung ließen sich jedoch nicht immer befriedigende Ergebnisse erzielen, insbesondere weil im gesamten Hirnbereich Glukose aufgenommen wird und aus diesem Grund kein ausreichender Kontrast zwischen normalem Gewebe und Tumorgewebe festgestellt werden kann und demnach eine ausreichende Abgrenzung des Tumorgewebes von gesundem Hirngewebe nicht möglich ist.

Wesentlich günstiger waren die Erfahrungen mit markierten Aminosäuren. Es konnten anfangs vielversprechende Ergebnisse mit der Aminosäure ¹¹C-Methionin erzielt werden. Diese Ergebnisse konnten belegen, dass zuverlässige Untersuchungen zur Feststellung von Hirntumoren sowie zum Verlauf einer Therapie durchführbar sind. Zunächst wurde angenommen, dass eine gesteigerte Proteinsynthese im Tumorgewebe die Ursache für die erhöhte Aminosäurekonzentration darstellt, aber momentan wird vielmehr angenommen, dass der Auslöser eine Veränderung des Aminosäuretransports ist. Eine solche Änderung des Aminosäuretransports kann aber nicht nur mit den normalen physiologischen Aminosäuren durchgeführt werden, sondern-auch mit Aminosäurederivaten, welche selber nicht für die Proteinsynthese verwendet werden können.

Die Untersuchungen mit ¹¹C-Methionin wiesen jedoch den praktischen Nachteil auf, dass die Halbwertzeit des ¹¹C von 20 Minuten sehr kurz ist und die PET-Untersuchungen mit ¹¹C-Methionin (und naturgemäß mit anderen mit ¹¹C markierten Verbindungen) nur in oder direkt an einem Institut durchgeführt werden kann, wo der kurzlebige Positronenstrahter ¹¹C hergestellt wird.

Um den Nachteil der Kurzlebigkeit von ¹¹C zu überwinden, wurden weitere Versuche mit ¹⁸Fmarkierten Aminosäuren durchgeführt. Die Halbwertzeit von ¹⁸F liegt mit 110 Minuten im Vergleich zu ¹¹C wesentlich günstiger. Eine solche Halbwertzeit erlaubt eine Herstellung in einer zentralen Einrichtung mit anschließendem Transport zu anderen Einrichtungen und Arztpraxen. Es liegen mittlerweile Erfahrungen mit 4-[¹⁸F]-Fluor-L-prolin, O-(2-[¹⁸F]Fluoroethyl)-L-tyrosin , 1-Amino-3-[¹⁸F]fluorcyclobutancarbonsäure und 3-[¹⁸F]Fluor-α-methyl-L-tyrosin vor. Die Verbindung O-(2-[¹⁸F]Fluorethyl)-L-tyrosin hat sich als sehr geeignet erwiesen und wird in der klinischen Praxis auch bereits erprobt (Weber W.A. et al.; Eur. J. Nucl. Med. 2000; 27: 542 -549).

Die Herstellung von O-(2-[¹⁸F]Fluoroethyl)-L-tyrosin wurde bislang mit einem relativ aufwendigen Verfahren durchgeführt (Wester H.J. et al.; J. Nucl. Med. 1999; 40: 205 - 212). Dabei wird in einem ersten Schritt [¹⁸F]Fluorethyltosylat hergestellt, und in einem zweiten Schritt wird ungeschütztes L-Tyrosin (als Di-Kaliumsalz) hiermit umgesetzt.
Ein Nachteil dieser Herstellung ist die Notwendigkeit, das ¹⁸F-Fluorierungsreagenz vor der weiteren Umsetzung chromatographisch zu reinigen.

Es war eine Aufgabe der vorliegenden Erfindung eine einfachere Herstellung von O-(2-[¹⁸F]Fluorethyl)-L-tyrosin zur Verfügung zu stellen.

Es war eine weitere Aufgabe der vorliegenden Erfindung, Ausgangsverbindungen zur Herstellung von O-(2-[¹⁸F]Fluorethyl)-L-tyrosin zur Verfügung zu stellen.

Die Erfindung betrifft zunächst neue Ausgangsverbindungen für die Herstellung von O-(2-[¹⁸F]-Fluorethyl-L-tyrosin ([¹⁸F] FET) gemäß der Formel (1): 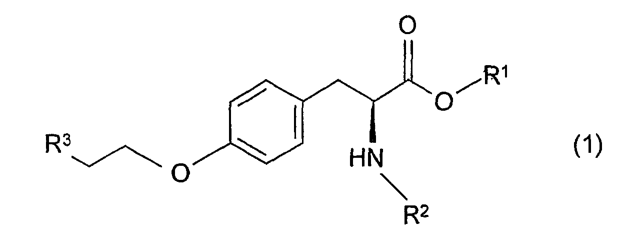 wobei R¹ eine geeignete Schutzgruppe für die Carboxylgruppe und R² eine geeignete Schutzgruppe für die Aminogruppe darstellt.

Die Gruppe R³ ist eine geeignete Abgangsgruppe für die Fluorierung des Ausgangsproduktes der Formel (1) zu der entsprechenden fluorierten Verbindung.

Geeignete Gruppen R¹ sind solche, welche die Carboxylgruppe unter den jeweiligen Reaktionsbedingungen schützen. Wie später im Rahmen der Beschreibung des erfindungsgemäßen Verfahrens näher beschrieben wird, sieht eine bevorzugte Ausführungsform vor, das Verfahren in einem dipolar aprotischen Lösungsmittel durchzuführen. Die Gruppe R¹ soll in dieser bevorzugten Ausführungsform des Verfahrens die Aufgabe erfüllen, geradezu unter den Bedingungen der nukleophilen ¹⁸F-Fluorierung nicht abgespalten zu werden. Es ist erwünscht, dass die Gruppe R¹ in einem letzten Schritt des Verfahrens zusammen mit der Gruppe R² mit wenig Aufwand unter aciden Bedingungen abgespalten werden kann,

Solche geeigneten Gruppen R' sind zum Beispiel solche Reste, die unter stark sauren Bedingungen auch in einem organischen, nicht wässrigen Lösungsmittel abgespalten werden können.

Gruppen R¹ gemäß der Erfindung sind die Mehylthiomethylgruppe, die Tetrahydrofuranylgruppe, die Diphenylmethylgruppe, die para-MethoxybenZylgruppe, die Piperonylgruppe und die tert-Butylgruppe.

Bevorzugt für die Gruppe R¹ ist die tert-Butylgruppe.

Geeignete Gruppen R² sind solche, welche die Aminogruppe der Aminosäure unter den jeweiligen Reaktionsbedingungen schützen. Wie später im Rahmen der Beschreibung des erfindungsgemäßen Verfahrens näher beschrieben wird, sieht eine bevorzugte Ausführungsform vor, das Verfahren in einem dipolar aprotischen Lösungsmittel durchzuführen. Die Gruppe R² soll in dieser bevorzugten Ausführungsform des Verfahrens die Aufgabe erfüllen, geradezu unter den Bedingungen der nukleophilen ¹⁸F-Fluoriuerung die Schutzwirkung auszuüben und eine Racemisierung der L-Aminosäure zu verhindern. Es ist erwünscht, dass die Gruppe in einem letzten Schritt des Verfahrens mit wenig Aufwand abgespalten werden kann.

Solche Gruppen R² sind Arylalkylreste.

Eine bevorzugte Gruppe R² ist die Triphenylmethylgruppe.

Die Gruppe R³ stellt eine geeignete Abgangsgruppe dar. Diese-Gruppe wird im eigentlichen Fluorierungsverfahren durch Fluor-18 substituiert.

Gruppen R³ im Rahmen der Verbindung sind p-Tosyloxy, Methansulfonyloxy, Trifluormethansulfonyloxy und Brom.

Eine bevorzugte Gruppe R³ ist die p-Tosyloxy-Gruppe.

Es hat sich herausgestellt, dass die Verbindungen der Formel (1) in kristalliner Form vorliegen sollten, was ihre Handhabung und Mengendosierung erheblich vereinfacht.

Die Verbindungen der Formel (1) sind besonders geeignet als Ausgangsverbindung zur Herstellung von O-(2-[¹⁸F]Fluoroethyl)-L-tyrosin.

Das erfindungsgemäße Verfahren zur Herstellung von O-(2-[¹⁸F]Fluorethyl)-L-tyrosin sieht vor, dass eine Ausgangsverbindung der Formel (1) in einem ersten Schritt ¹⁸F-fluoriert wird und in einem zweiten Schritt die Schutzgruppen R¹ und R² abgespalten werden, wobei die gewünschte Endverbindung O-(2-[¹⁸F]Fluorethyl)-L-tyrosin erhalten wird.

Zur Fluorierung wird eine Verbindung der Formel (1) in Gegenwart eines Phasentransfer-Systems, z.B, der Formel R₄N⁺¹⁸F umgesetzt, wobei R eine Alkylgruppe mit 3 bis 6 C-Atomen, vorzugsweise n-Butyl darstellt. ¹⁸F wird durch-ein an sich bekanntes Verfahren hergestellt, indem mit ¹⁸O angereichertes Wasser durch eine (p,n)-Kernreaktion in einem Zyklotron in [¹⁸F]Fluorid umgewandelt wird.

Die Fluorierung wird mit Hilfe eines Phasentransfer-Katalysators durchgeführt. Als solche Phasentransfer-Katalysatoren kommen grundsätzlich Katalysatoren wie [K⊂2.2.2]₂CO₃ und (TBA⁺HCO₃⁻) in Betracht.

Es hat sich herausgestellt, dass bei Durchführung des Fluorierungsschrittes mit [K⊂2.2.2]₂CO₃ als Katalysator eine Ausbeute von etwa 10 % erzielt werden kann.

Es hat sich weiter herausgestellt, dass dieses Ergebnis bei der Durchführung des Fluorierungsschrittes mit (TBA⁺HCO₃⁻) als Katalysator verbessert werden kann. Wenn als Ausgangsverbindung der Herstellung von O-(2-[¹⁸F]Fluorethyl)-L-tyrosin die besonders bevorzugte Ausgangsverbindung N-Trityl-O-(2-Tosyloxyethyl)-L-tyrosin tert-butylester verwendet wird, wird mit (TBA⁺HCO₃⁻) als Katalysator eine radiochemische Ausbeute von über 80 % erzielt. Dies stellt ein überraschend gutes Ergebnis dar.

Als Lösungsmittel für die nukleophile ¹⁸F-Fluorierung kommen die üblichen organischen Lösungsmittel in Betracht. Es hat sich herausgestellt, dass die Durchführung mit einem aprotischen Lösungsmittel wie z.B., Acetonitril, N,N-Dimethylacetamid, N,N-Dimethylformamid, Dimethlylsulfoxid und Hexamethylphosphorsäuretriamid besonders günstig abläuft wobei Acetonitril aufrgund seiner physikalisch-chemischen Eigenschaften besonders geeignet ist.

Der erste Schritt wird üblicherweise bei einer Temperatur von etwa 85°C durchgeführt. Eine Dauer von 5 Minuten ist üblicherweise ausreichend für die Durchführung.

Nach Ablauf des ersten Schrittes, wie vorstehend erwähnt, nach in etwa 5 Minuten wird die Entschützung durchgeführt. Es ist nicht erforderlich, vorher eine Vorreinigung vorzunehmen, und die Tatsache, dass der zweite Schritt der Entschützung im gleichen Behälter abläuft wie der erste Schritt, und es sich demnach um ein Eintopf-Verfahren handelt, betrifft einen attraktiven Aspekt der Erfindung.

Die Entschützung selber wird durch Hinzufügen einer starken Säure ausgelöst, es kommt hier auf eine Säure an, die auch in einem nicht-wässrigen System wie z.B. Dichlormethan eingesetzt werden kann. Besonders gut läuft die Entschützung mit Trifluoressigsäure ab.

Nach der Entschützung liegt die Verbindung O-(2-[¹⁸F]Fluoroethyl)-L-tyrosin in unreiner Form vor.

Der letzte Reinigungsschritt der Endverbindung O-(2-[¹⁸F]Fluoroethyl)-L-tyrosin wird mit üblichen Mitteln und Methoden vorgenommen. Die Abtrennung der markierten Aminosäure kann sehr günstig mittels Festphasenextraktion aus der organischen Phase erfolgen. Dabei bietet sich die Anwendung von Silikagel und Aluminiumoxid an. Wenn diese Extraktion mit Silikagel und Aluminiumoxid durchgeführt wird, wird üblicherweise an der Festphase adsorbierte Säure durch einen geeigneten Elutionsschritt weitgehend entfernt. Hier kommt es nicht besonders darauf an, welches Lösungsmittel für die Elution-genommen wird, es spielt für die Auswahl des Lösungsmittels eine Rolle, welche Säure verwendet wurde und welche Festphasenkombination zum Einsatz kam. Im allgemeinen hat sich gezeigt, dass ein Gemisch aus n-Pentan und Diethylether für diesen Elutionschritt, speziell bei Verwendung von Trifluoressigsäure sehr geeignet ist.

Nach der Elution können die der Festphase anhaftenden Lösungsmittelreste mit einem Inertgas, wie z.B. Stickstoff oder Argon, verdampft und ausgetragen werden und die markierte Aminosäure mit einer geeigneten Pufferlösung extrahiert werden. Es wird üblicherweise eine solche Pufferlösung eingesetzt, die ein unmittelbare HPL-Chromatographie ermöglicht.

Es ist der große Vorteil des erfindungsgemäßen Verfahrens, dass die markierte Aminosäure mit relativ wenig Aufwand in einem Eintopf-Verfahren hergestellt werden kann. Das Verfahren erlaubt die Herstellung unter Vermeidung einer Racemisierung. Der Einsatz von [¹⁸F]Fluorid garantiert ein trägerarmes Endprodukt, was für eine Anwendung am Patienten notwendig ist. Es ist der Vorteil des erfindungsgemäßen Verfahrens, dass eine Herstellung einer markierten Aminosäure mit einer molaren Aktivität von mehr als 18,5 GBq/µmol ohne weiteres möglich ist.

Die Herstellung der Ausgangsverbindungen der Formel (1) kann mit an sich bekannten Verfahren durchgeführt werden. Man kann bei der Herstellung von der Aminosäure L-Tyrosin selbst ausgehen und die weiteren Reaktionsmittel nach dem gewünschten Endprodukt auswählen.

Wenn eine Ausgangsverbindung der Formel (1) hergestellt werden soll, welche als Gruppe R¹ eine tert-Butylgruppe aufweist, ist es geeignet, von einer Verbindung L-Tyrosin tertbutylester auszugehen, da diese Verbindung kommerziell erhältlich ist. Ansonsten wird L-Tyrosin in einem ersten Schritt mit einem Reagens zusammengeführt, dass in der Lage ist, eine Reaktion mit der Carboxylgruppe einzugehen unter Bildung einer Verbindung der Formel (2):

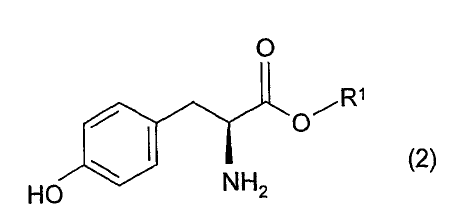

Dabei hat R¹ die gleiche Bedeutung wir bei der Beschreibung der erfindungsgemäßen Ausgangsverbindungen beschrieben. Geeignete Reagentien zur Durchführung der Reaktion sind insbesondere Alkohole, wie z.B. tert-Butanol oder das Olefin Isobuten.

In einem zweiten Schritt reagiert die Verbindung der Formel (2) mit einer Verbindung R²X wobei eine Verbindung der Formel (3) 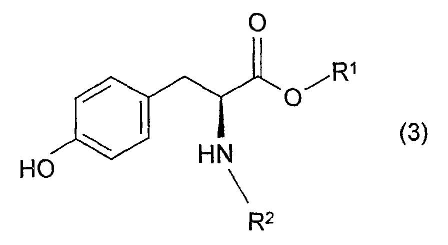 gebildet wird. Die Gruppe R² hat hierbei die gleiche Bedeutung wie in Zusammenhang mit der Beschreibung der erfindungsgemäßen Ausgangsverbindungen beschrieben wurde. X stellt ein Halogen dar, vorzugsweise Chlor.

In einem nächsten Schritt wird die Verbindung der Formel (3) in eine Verbindung der formel (1) umgewandelt, wobei in diesem letzten Schritt an der Hydroxylgruppe des aromatischen Ringes eine R³-C₂N₄- Gruppe eingeführt wird. Geeignete Reagentien für diesen letzten Schritt sind 1,2-bifunictionelle Ethylenderivate, wie z.B. Ethylenglykoldi(p-toluolsulfonat) und 2-Bromethanol.

Es ist eine bevorzugte Ausführungsform der Verbindungen der Formel (1), dass die Gruppe R3 eine Tosyloxygruppe darstellt, für diese bevorzugte Ausführung wird der letzte Schritt mit Ethylenglykol-di(p-toluolsulfonat) durchgeführt.

Die Erfindung wird weiter anhand der nachfolgenden Beispiele näher beschrieben:

### Beispiel 1

Herstellung von O-(2-Tosyloxyethyl)-N-(triphenylmethyl)-Ltyrosin-tert butylester:
1. Stufe N-Triphenylmethyl-L-tyrosin tert butylester
   L-Tyrosin tert butylester* wird bei Raumtemperatur in DMF, in Gegenwart von Triethylamin, mit einer äquimolaren Menge Triphenylmethylchlorid umgesetzt. Durch Vermischung der DMF-Phase mit der 4-fachen Menge Eis wird das Rohprodukt präzipitiert und aus Ethanol umkristallisiert.
   Ausbeute: 70 % Fp. 171°C
   (*kommerziell erhältlich)
2.Stufe O-(2-Tosyloxyethyl)-N-(triphenylmethyl)-L-tyrosin-tert butylester:
   Äquimolare Mengen N-Triphenylmethyl-L-tyrosin tert butylester und Ethylenglykoldi-(p-toluolsulfonat) werden in Aceton gelöst. In Gegenwart einer Base wird das Eduktgemisch zwei Tage bei Raumtemperatur intensiv gerührt. Nach Filtration und Eindampfen der Rohproduktlösung erfolgt die Reinigung des Produktes säulenchromatographisch an Kieselgel mit einem Laufmittel bestehend aus niedrig siedendem Petrolether und Ethylacetat.
   Ausbeute: 60 % Fp. 54...64°C

### Beispiel 2.

### Herstellung O-(2-[18F]-Fluorethyl-L-Tyrosin)

Das ¹⁸F-haltige Wasser wird in Gegenwart von 40 µmol Tetra-n-butylammoniumhydrogencarbonat zur Trockene eingedampft. Anschließend wird eine Lösung von 25 mg (37 µmol) N-Trityl-O-(2-Tosyloxyethyl)-L-tyrosin-tert.-butylester in 0,5 ml Acetonitril zugegeben und die Lösung 5 min zum Sieden erhitzt. Unter vermindertem Druck wird das Lösungsmittel abgedampft, bei einer Temperatur von etwa 20°C 1 ml einer Mischung von Trifluoressigsäure/Triethylsilan/Dichlormethan (1/0,4/2) (v/v/v) zugesetzt und 10 min gerührt. Nach Verdünnung mit 9 ml Dichlormethan wird die Lösung nacheinander durch eine Silikagel- und eine Aluminiumoxid-Kartusche (je 1 g) geleitet, Die Kartuschen werden mit 10 ml eines Lösungsmittelgemisches aus n-Pentan/Diethytether (1/1) gewaschen. Durch einen Inertgasstrom wird das restliche Lösungsmittel verdampft, das [¹⁸F]FET-Rohpradukt mit Hilfe einer 35 mmolaren Trinatriumphosphatlösung eluiert und durch *reversed phase* Hochdruckflüssigkeitschromatographie gereinigt. Die [¹⁸F]FET-Produktfraktion wird durch einen Kationenaustauscher (LiChrolut SCX, H⁺-Form, 1g) geleitet, danach mit Sterilwasser gespült und die ¹⁸F-markierte Aminosäure mit einem Natriumphosphatpuffer physiologischer Konzentration eluiert und das Eluat sterilfiltriert.

## Patentansprüche

1. L-Tyrosinderivate der Formel (1) 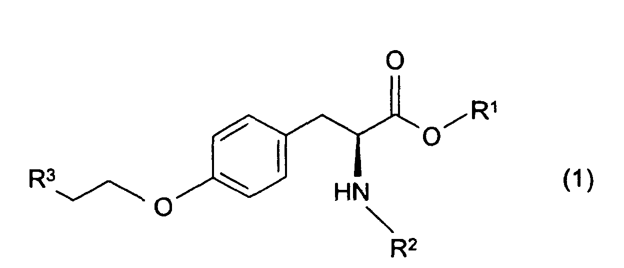 wobei R¹ eine geeignete Schutzgruppe für die Carboxygruppe darstellt, R² eine geeignete Schutzgruppe für die Aminogruppe darstellt und R³ eine geeignete Abgangsgruppe darstellt,
wobei R' eine Methylthiomethylgruppe, eine Tetrahydrofuranylgruppe, eine Diphenylmethylgruppe, eine para-Methoxybenzylgruppe, eine Piperonylgruppe oder eine tert-Butylgruppe darstellt,
R² eine Arylalkylgruppe darstellt und
R³ p-Toluolsulfonyloxy, Methansulfonyloxy, Trifluormethansulfonyloxy oder Brom darstellt.

2. Tyrosinderivate gemäß Anspruch 1, worin R¹ eine tert-Butylgruppe darstellt, R² eine Triphenylmethyl-Gruppe darstellt und R³ eine Toluolsulfonyloxygruppe darstellt.

3. Verfahren zur Herstellung von O-(2-[¹⁸F]-Fluorethyl-L-tyrosin), wobei eine Ausgangsverbindung der Formel (2) 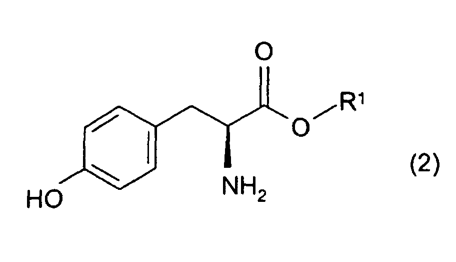 mit R²X zu einer Verbindung der Formel (3) 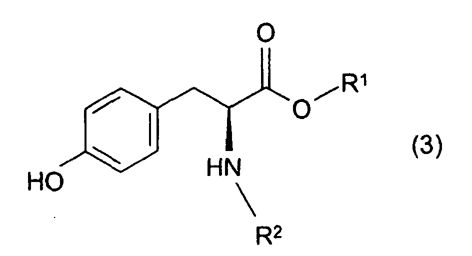 umgesetzt wird und in einem zweiten Schritt die Verbindung der Formel (3) in eine Verbindung der Formel (1) umgewandelt wird, 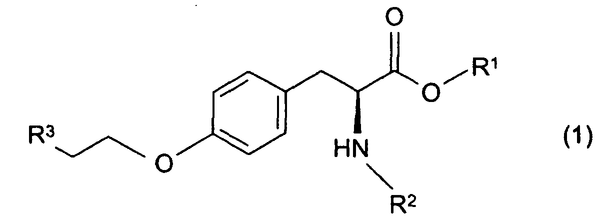 und die Verbindung der Formel (1 ) in einem dritten Schritt mit einem Tetraalkylammonium[18F]fluorid der Formel
R₄N⁺¹⁸F
oder einem Kryptat der Formel
[K⊂2.2.2]¹⁸F
in Gegenwart eines Phasentransfer-Katalysators,
in einem aprotischen Lösungsmittel,
zu der Verbindung der Formel (5) 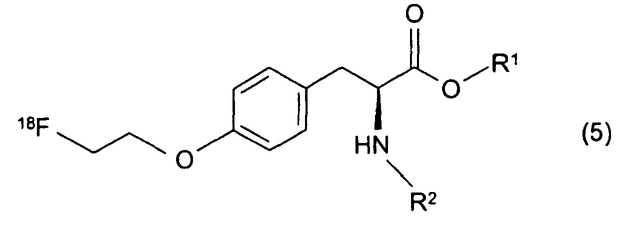 reagiert und
in einem weiteren Schritt in einem aciden System die Gruppen R¹ und R² abgespalten werden und das entstandene Produkt O-(2-[¹⁸F]-Fluorethyl-L-tyrosin) anschließend gereinigt wird,
wobei
R¹, R² und R³ die gleiche Bedeutung wie in Anspruch 1 haben
X ein Halogen ist und
R eine Alkylgruppe mit 3 bis 6 C-Atomen, insbesondere eine n-Butylgruppe darstellt.

4. Verfahren gemäß Anspruch 3,
wobei der Phasentransfer-Katalysator Tetrabutylammonium hydrogencarbonat ist.

5. Verfahren nach einem der Ansprüche 3 oder 4, wobei das aprotische Lösungsmittel ausgewählt ist aus der Gruppe bestehend aus Acetonitril, N,N-Dimethylacetamid, N,N-Dimethylformamid, Dimethylsulfoxid und Hexamethylphosphorsäuretriamid.

6. Verfahren nach einem der Ansprüche 3 bis 5, wobei das acide System im letzten Verfahrensschritt Trifluoressigsäure in Dichlormethan umfasst.

## Claims

1. L-tyrosine derivatives of formula (1) 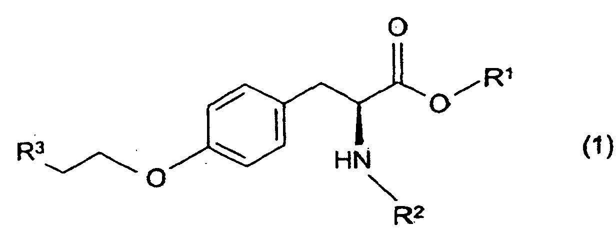 wherein R¹ denotes a suitable protective group for the carboxy group, R² denotes a suitable protective group for the amino group and R³ denotes a suitable leaving group,
wherein R¹ denotes a methylthiomethyl group, a tetrahydrofuranyl group, a diphenylmethyl group, a para-methoxybenzyl group, a piperonyl group or a tert-butyl group,
R² denotes an arylalkyl group and
R³ denotes p-tosyloxy, methanesulfonyloxy, trifluoromethanesulfonyloxy or bromine.

2. Tyrosine derivative according to claim 1, wherein R¹ denotes a tert-butyl group, R² denotes a triphenylmethyl group and R³ denotes a tosyloxy group.

3. Method for the manufacture of O-(2-[¹⁸F]-fluoroethyl-L-tyrosine), wherein a parent compound of formula (2) 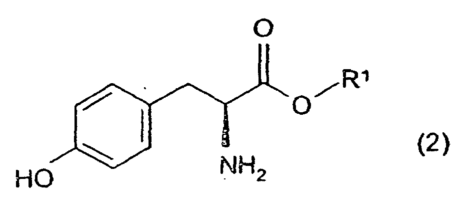 is converted with R²X to form a compound of formula (3) 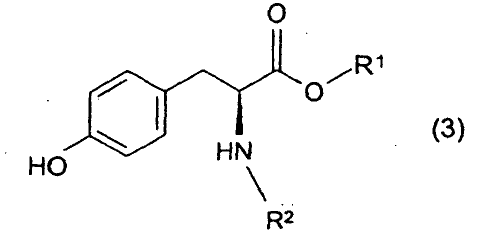 and in a second stage, the compound of formula (3) is converted into a compound of formula (1), 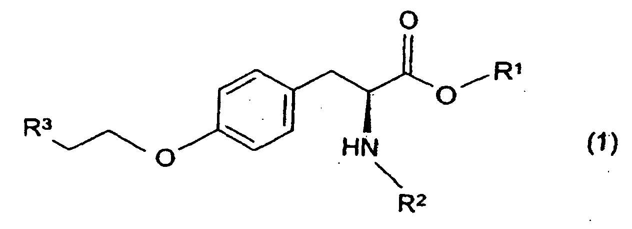 and in a third stage, the compound of formula (1) reacts with a tetraalkylammonium [¹⁸F]-fluoride of formula
R₄N⁺¹⁸F
or with a cryptate of formula
[K⊂2.2.2]¹⁸F
in the presence of a phase-transfer catalyst, in an aprotic solvent, to form the compound of formula (5) 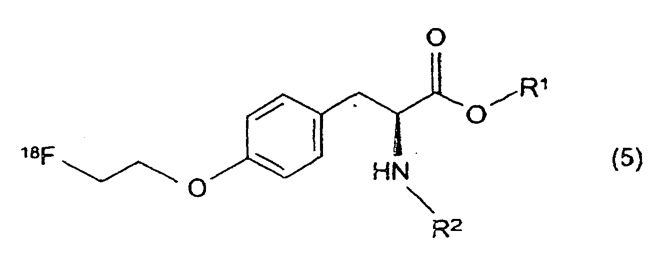 and
in a further stage, in an acidic system, the groups R¹ and R² are split off and the resulting product O-(2-[¹⁸F]-fluoroethyl-L-tyrosine) is then purified, wherein
R¹, R² and R³ have the same meaning as in claim 1, X is a halogen and
R denotes an alkyl group with 3 to 6 C-atoms, especially an n-butyl group.

4. Method according to claim 3,
wherein the phase-transfer catalyst is tetrabutylammonium hydrogen carbonate.

5. Method according to any one of claims 3 or 4,
wherein the aprotic solvent is selected from the group consisting of acetonitrile, N,N-dimethylacetamide, N,N-dimethylformamide, dimethylsulfoxide and hexamethylphosphoryl triamide.

6. Method according to any one of claims 3 to 5,
wherein the acidic system in the last stage of the method comprises trifluoroacetic acid in dichloromethane.

## Revendications

1. Dérivés de L-tyrosine de formule (1) : 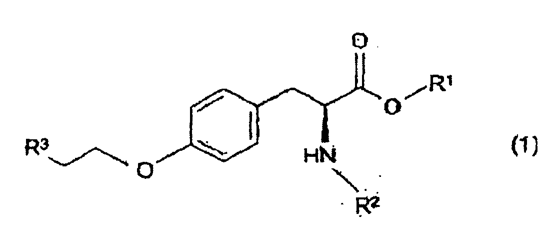 dans laquelle R¹ représente un groupe protecteur approprié pour le groupe carboxyle, R² représente un groupe protecteur approprié pour le groupe amine et R³ représente un groupe partant approprié.
R¹ représentant un groupe méthylthiométhyle, un groupe tétrahydrofuranyle, un groupe diphénylméthyle, un groupe paraméthoxybenzyle, un groupe pipéronyle ou un groupe tert-butyle,
R² représentant un groupe arylalkyle et
R³ représentant un radical p-toluènesulfonyloxy, méthanesulfonyloxy, trifluorométhanesulfonyloxy ou brome.

2. Dérivés de la tyrosine selon la revendication 1, dans lesquels R¹ représente un groupe tert-butyle, R² représente un groupe triphénylméthyle et R³ représente un groupe p-toluènesulfonyloxy.

3. Procédé de production de O-(2-[¹⁸F]-fluoroéthyl)-L-tyrosine, dans lequel un composé de départ de formule (2) 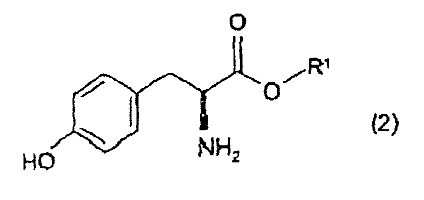 est réagi avec R²X en un composé de formule (3) 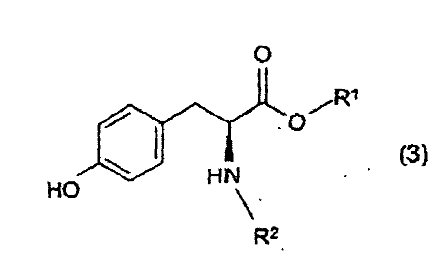 et dans une deuxième étape le composé de formule (3) est transformé en un composé de formule (1), 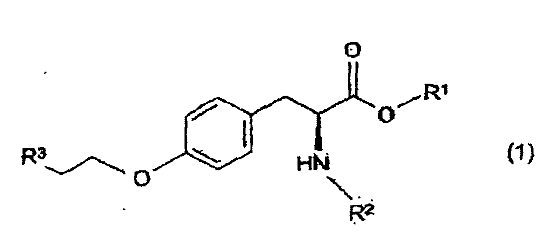 et le composé de formule (1) est réagi dans une troisième étape avec un [¹⁸F]-fluorure de tétraalkylammonium de formule
R₄N⁺¹⁸F
ou un cryptate de formule
[Kc2.2.2)¹⁸F
en présence d'un catalyseur de transfert de phase, dans un solvant aprotique en un composé de formule (5) 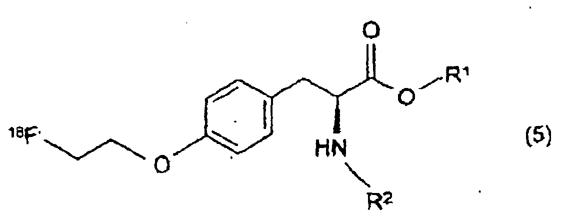 et dans une étape supplémentaire, les groupes R¹ et R² sont éliminés dans un système acide et le produit O-(2-[¹⁸F]-fluoroéthyl)-L-tyrosine formé est ensuite purifié, R¹, R² et R³ ayant la même signification que dans la revendication 1, X étant un halogène et R représentant un groupe alkyle ayant 3 à 6 atomes de C, en particulier un groupe n-butyle.

4. Procédé selon la revendication 3, dans lequel le catalyseur de transfert de phase est l'hydrogénocarbonate de tétrabutylammonium

5. Procédé selon l'une des revendications 3 ou 4, dans lequel le solvant aprotique est choisi dans le groupe composé de l'acétonitrile, du N,N-diméthylacétamide, du N,N-diméthylformamide, du diméthylsulfoxyde et de l'hexaméthylphosphorotriamide.

6. Procédé selon l'une des revendications 3 à 5, dans lequel le système acide dans la dernière étape du procédé contient l'acide trifluoroacétique dans du dichlorométhane.
